(19) Europäisches Patentamt
European Patent Office
Office européen des brevets

(11) Numéro de publication: **0 240 431 B1**

(12) **FASCICULE DE BREVET EUROPEEN**

(45) Date de publication de fascicule du brevet: **27.11.91**

(51) Int. Cl.$^5$: **C07C 45/51**, C07C 47/21, C07C 47/225

(21) Numéro de dépôt: **87400724.8**

(22) Date de dépôt: **02.04.87**

(54) **Procédé de préparation de composes carbonyles éthyléniques.**

(30) Priorité: **03.04.86 FR 8604769**

(43) Date de publication de la demande:
**07.10.87 Bulletin 87/41**

(45) Mention de la délivrance du brevet:
**27.11.91 Bulletin 91/48**

(84) Etats contractants désignés:
**AT BE CH DE FR GB IT LI LU NL SE**

(56) Documents cités:
**EP-A- 0 127 536**
**FR-A- 1 554 805**
**FR-A- 1 576 228**

(73) Titulaire: **RHONE-POULENC SANTE**
**20, avenue Raymond Aron**
**F-92160 Antony(FR)**

(72) Inventeur: **Chabardes, Pierre**
**24 rue Jeanne d'Arc**
**F-69110 Sainte Foy Les Lyon(FR)**

(74) Mandataire: **Le Pennec, Magali et al**
**RHONE-POULENC RORER SA, Direction des Brevets, 20 Avenue Raymond Aron**
**F-92165 Antony Cédex(FR)**

**EP 0 240 431 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un nouveau procédé de préparation de composés carbonylés par isomérisation d'alcools α-acétyléniques.

Il est connu d'isomériser en milieu acide des arylalcynylcarbinols en composés carbonylés éthyléniques [K.H. MEYER et coll., Ber. 55, 819-823 (1922); M. BADOCHE, Bull. Soc. Chim. (France), (4), 43, 340 (1928) ; W.S. Mac GREGOR, J. Amer. Chem. Soc., 70, 3953 (1948) ; N. HAGIHARA, CHEM. Abstr, 45, 8997 g (1951) ; E.T. CLAPPERTON et coll., J. Amer. Chem. Soc., 72, 2501-2 (1950)]. Il est connu également de soumettre à l'action de catalyseurs acides et en phase vapeur des alcools acétyléniques secondaires ou tertiaires en vue d'obtenir des mélanges d'aldéhydes insaturés, de cétones insaturées et d'hydrocarbures éthyléniques acétyléniques [brevet américain US 2 524 865 ; E.D. BERGMANN, J. Amer. Chem. Soc., 73, 1218-1220 (1951)]. Il est connu également d'effectuer l'isomérisation du méthylbutynol en prénal en présence de catalyseur à base d'oxyde de molybdène déposé sur silice en opérant à 350-400 °C pour obtenir une bonne sélectivité (brevet russe SU 827 477).

Cependant ces procédés sont difficilement utilisables industriellement.

Il est aussi connu, d'après le brevet français FR 1 554 805 et son addition FR 95548, d'isomériser catalytiquement les alcools acétyléniques en composés carbonylés éthyléniques par chauffage en phase liquide en présence d'un catalyseur à base d'un métal choisi dans le groupe constitué par le vanadium, le niobium, le molybdène, le tungstène et le rhénium, et plus particulièrement un dérivé métallique comportant un enchaînement ayant l'une des formules suivantes :

-M=O       -O-M=O

-M⟵ O=       -O-M⟵ O=

M représentant l'atome de métal, tel que par exemple l'orthovanadate de cyclohexyle.

Cependant, il n'est pas possible d'atteindre un taux de transformation complet de l'alcool α-acétylénique, à moins d'opérer en milieu très dilué et en présence de silanols qui sont des produits coûteux.

Il a maintenant été trouvé, et c'est ce qui fait l'objet de la présente invention, que les alcools acétyléniques de formule générale :

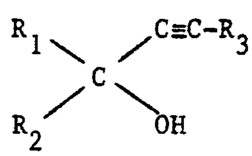

(I)

peuvent être isomérisés directement en composés carbonylés de formule générale :

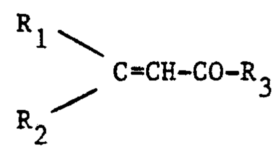

(II)

par chauffage en phase liquide en présence d'un système catalytique constitué d'un dérivé du titane et d'un dérivé du cuivre ou de l'argent et, éventuellement en présence d'un ester d'acide minéral ou organique, ou d'un anhydride d'acide, ou de préférence, d'un acide organique.

Dans les formules générales (I) et (II), $R_1$, $R_2$ et $R_3$, identiques ou différents, représentent chacun un atome d'hydrogène ou un radical aliphatique saturé ou non saturé, un radical cycloaliphatique saturé ou non saturé, un radical aromatique ou un radical arylaliphatique, ou bien $R_1$ et $R_2$ forment ensemble un radical divalent et $R_3$ représente un atome d'hydrogène ou un radical aliphatique saturé ou non saturé, un radical

cycloaliphatique saturé ou non saturé, un radical aromatique ou un radical arylaliphatique, étant entendu que les radicaux aliphatiques, cycloaliphatiques, aromatiques ou arylaliphatiques peuvent être substitués par un ou plusieurs substituants, identiques ou différents, choisis parmi les atomes d'halogène et les radicaux hydroxy, alcoxy, phénoxy, acyle ou alcyloxy.

De préférence $R_1$, $R_2$ et $R_3$ contiennent ensemble 2 à 30 atomes de carbone et l'un au moins des radicaux $R_1$ et $R_2$ est un atome d'hydrogène ou un radical alcoyle saturé ou non saturé éventuellement substitué comportant 1 à 15 atomes de carbone.

D'un intérêt tout particulier sont les produits de formule générale (I) ou (II) dans laquelle $R_3$ représente un atome d'hydrogène.

Le procédé selon l'invention est particulièrement utile pour isomériser le méthylbutynol en prénal ou le déhydrolinalol en citral ; le prénal et le citral étant des intermédiaires particulièrement intéressants pour la préparation de la vitamine A ou de la vitamine E.

Pour la mise en oeuvre du procédé selon l'invention, il est nécessaire d'utiliser un système catalytique contenant simultanément le dérivé du titane et le dérivé du cuivre ou de l'argent et éventuellement un acide organique ou un dérivé (ester, anhydride) ou un ester inorganique.

Le dérivé du titane est choisi parmi les dérivés du titane au degré d'oxydation II, III ou IV.

Les dérivés du titane qui conviennent particulièrement bien sont les dérivés de formule générale $TiR_4$ dans laquelle les symboles R, identiques ou différents, représentent un atome d'halogène ou un radical OR' ou OCOR' (dans lequel R' représente un radical alcoyle contenant 1 à 20 atomes de carbone), ou un radical $-OSiR_1R_2R_3$ (dans lequel les symboles $R_1$, $R_2$ et $R_3$, identiques ou différents, représentent un radical alcoyle tel que méthyle ou éthyle ou un radical aryle tel que phényle). Peuvent être également avantageusement utilisés les chélates du titane tels que les chélates du titane avec l'acétylacétone, la benzoylacétone, les acétylacétates d'alcoyle ou l'aldéhyde salicylique. Les dérivés du titane qui sont utilisés pour la mise en oeuvre du procédé selon l'invention sont, par exemple, les titanates d'alcoyle de formule $Ti(OR_5)_4$ tel que le titanate de butyle ou d'isopropyle, les sels d'acides carboxyliques de formule $Ti(OCOR_6)_4$ ou les dérivés mixtes de formule $Ti(OR_5)_{4-n}$ $(OCOR_6)_n$ ou $Ti(OR_5)_n$ $(OSiR_1R_2R_3)_{4-n}$ ou $Ti(OR_5)_2$ (agent chélatant)$_2$ ou $TiCl_2$ $(OCOR_6)_2$ et les polytitanates. Ces dérivés du titane sont décrits en particulier dans "The Organic Chemistry of Titanium" par FELD et COWE, LONDON BUTTERWORTHS (1965).

Parmi les dérivés du titane à différents degrés d'oxydation, qui peuvent être utilisés pour la mise en oeuvre du procédé selon l'invention, peuvent être cités, par exemple, le trichlorure de titane, les oxyhalogénures de titane tels que $TiOCl_2$, TiOCl, TiOBr, les oxytitanes, tels que le sulfate de titanyle, le dichlorure de titanocène, l'acétylacétonate de titanyle, $TiO(SbF_6)_2$, $TiO(TiF_6)$, $TiO[(C_2H_5)_4N]_2Cl_4$, TiO (phtalocyanine), les dérivés du titane de formule $O = T(X)_2$ dans laquelle X représente un radical OR' ou OCOR' défini comme précédemment, et, plus généralement les dérivés du titane qui sont décrits par M. Bottrill et coll., Comprehensive Organic Chemistry, Vol. 8, p. 332-426 (1982) édité par Wilkinson, Pergamon Press ou par R.J.H. Clark, The Chemistry of Titanium, Zirconium et Hafnium, Pergamon Texts in Inorganic Chemistry, Vol. 19, (1973), Pergamon Press.

Les dérivés du titane peuvent éventuellement être préparés in situ en introduisant dans le mélange réactionnel les réactifs nécessaires à leur formation.

Parmi les dérivés du titane qui conviennent particulièrement bien peuvent être cités le titanate de butyle ou le titanate d'isopropyle.

Les dérivés du cuivre ou de l'argent sont choisis parmi les sels d'acides minéraux ou organiques ou les complexes métalliques. Des résultats particulièrement satisfaisants sont obtenus en utilisant le chlorure cuivreux, le chlorure cuivrique ou l'oxalate cuivrique ou le trifluoroacétate d'argent.

Les acides organiques, éventuellement utilisés sous forme d'ester ou d'anhydride sont choisis parmi les acides ou diacides aliphatiques saturés ou non saturés contenant 1 à 20 atomes de carbone (tels que les acides acétique, hexanoïque, heptanoïque, éthyl-2 hexanoïque, octanoïque, adipique, crotonique) et les acides ou diacides aromatiques (tels que les acides benzoïque, téréphtalique) éventuellement substitués (tel que les acides méthyl-4 benzoïque, méthoxy-4 benzoïque, phénoxy-4 benzoïque) ou les acides arylaliphatiques (acide phénylacétique). Il est possible également d'utiliser un ester d'énol tel que l'acétoxy-2 propène. Les esters inorganiques sont choisis parmi les esters de l'acide phosphorique (tel que le phosphate de tributyle) ou de l'acide sulfonique (tel que le paratoluènesulfonate de butyle).

Il est particulièrement avantageux de mettre en oeuvre le procédé selon la présente invention en présence d'un acide organique.

Généralement, pour une mole d'alcool acétylénique de formule générale (I) mis en oeuvre, on utilise 0,005 à 0,05 mole de dérivé du titane, 0,005 à 0,1 mole de dérivé du cuivre et 0,01 à 1 mole d'acide organique ou de son dérivé ou d'ester inorganique.

Généralement, le procédé selon l'invention est mis en oeuvre à une température comprise entre 80 et

180 °C, de préférence voisine de 130 °C, la réaction étant compète après 1 à 2 heures de chauffage. Il est possible d'opérer dans un solvant organique choisi parmi les hydrocarbures aliphatiques, alicycliques ou aromatiques éventuellement substitués par un ou plusieurs atomes d'halogène ou radicaux alcoxy, nitro, cyano tels que le dichloroéthane, le dichlorobenzène, l'anisole, le phénétole, le nitrobenzène, le dicyclohexyle ou le benzonitrile, les amides (N-méthylpyrrolidone) ou les cétones (cyclohexanone). Les acides organiques éventuellement sous forme d'ester ou d'anhydride peuvent aussi être utilisés comme solvants.

Lorsque l'on utilise un ester organique (acétate de butyle), il peut être avantageux d'opérer en présence d'un alcool (butanol) ou d'un silanol (triphénylsilanol) mais, dans ce cas, il importe que le rapport molaire du silanol au dérivé du titane soit inférieur à 4.

Le procédé selon la présente invention peut être mis en oeuvre en continu ou en discontinu. En fin de réaction le catalyseur peut être récupéré et utilisé à nouveau pour de nouvelles opérations d'isomérisation.

Le composé carbonylé éthylénique de formule générale (II) obtenu selon le procédé de la présente invention peut être isolé par tout moyen connu en soi, par exemple par distillation ou par l'intermédiaire d'une combinaison sulfitique ou bisulfitique dans le cas des aldéhydes. Pour certaines applications, il n'est pas nécessaire d'isoler le composé carbonylé éthylénique et l'ensemble des produits constituant le milieu réactionnel peut être utilisé directement pour réaliser des synthèses à partir du composé carbonylé éthylénique obtenu. Il en est ainsi, par exemple, pour la préparation de ionones à partir du citral qui est obtenu par isomérisation du déhydrolinalol.

Les exemples suivants, donnés à titre non limitatif, montrent comment l'invention peut être mise en pratique.

EXEMPLE 1

1) Dans un ballon tricol de 50 cm3 muni d'un réfrigérant, d'une arrivée d'argon et d'un septum pour prise d'échantillon et d'une agitation magnétique, on introduit, sous atmosphère d'argon :

- méthylbutynol : 12,8 g (152,2 m.moles)
- acide méthyl-4 benzoïque : 3,6 g (26,4 m.moles)
- dichlorobenzène : 19,6 g
- dicyclohexyle (étalon interne pour chromatographie) : 5,3 g
- titanate de butyle : 0,75 g (2,20 m.moles)
- chlorure cuivreux : 0,3 g (3,03 m.moles)

Le ballon est chauffé au moyen d'un bain d'huile dont la température est régulée à 130 °C. En 12 minutes, la température du mélange réactionnel passe de 50 à 126 °C et il se forme un précipité jaune. Au bout de 15 minutes, la température monte à 137 °C puis se stabilise à 125-126 °C. Après 1 heure d'agitation à cette température, le mélange réactionnel est refroidi à une température voisine de 20 °C.

L'analyse du produit brut de réaction par chromatographie en phase vapeur montre que :

- le taux de conversion du méthylbutynol est de 96,6 %
- le rendement en prénal par rapport au méthylbutynol transformé est de 90 %.

Le produit brut de réaction, qui est hétérogène, est distillé sous pression réduite (15 mm de mercure ; 2 kPa), en chauffant à une température maximale de 72 °C. On obtient ainsi :

- 13,2 g de distillat incolore dont l'analyse montre que le taux de conversion du méthylbutynol est de 96,3 % et que le rendement en prénal est de 86 % par rapport au méthylbutynol transformé
- 28,8 g de culot dont l'analyse montre qu'il contient 1 % de prénal.

2) Au culot obtenu précédemment (28,8 g), on ajoute 12,8 g de méthylbutynol puis on chauffe pendant 1 heure 30 minutes au moyen d'un bain d'huile dont la température est régulée à 130 °C.

L'analyse du produit brut de réaction par chromatographie en phase vapeur montre que :

- le taux de conversion du méthylbutynol est de 94 %
- le rendement en prénal par rapport au méthylbutynol transformé est de 90 %.

Le produit brut de réaction est distillé sous pression réduite (15 mm de mercure ; 2 kPa), en chauffant à une température maximale de 73 °C. On obtient ainsi :

- 13,9 g de distillat incolore dont l'analyse montre que le taux de conversion du méthylbutynol est de 94 % et que le rendement en prénal est de 89 % par rapport au méthylbutynol transformé.
- 27,35 g de culot dont l'analyse montre qu'il contient 1,1 % de prénal.

3) Au culot obtenu précédemment (27,35 g), on ajoute 12,8 g de méthylbutynol et 2,9 g de dichlorobenzène, puis on chauffe pendant 2 heures au moyen d'un bain d'huile dont la température est régulée à 130 °C.

L'analyse du produit brut de réaction par chromatographie en phase vapeur montre que le taux de transformation du méthylbutynol est de 88 % et que le rendement en prénal est de 91 % par rapport au

méthylbutynol transformé.

Le produit brut de la réaction est distillé sous pression réduite (20 mm de mercure ; 2,7 kPa), en chauffant à une température maximale de 72° C. On obtient ainsi :

- 12,4 g de distillat incolore dont l'analyse montre que le taux de transformation du méthylbutynol est de 87 % et que le rendement en prénal est de 82 % par rapport au méthylbutynol transformé.

L'analyse des trois distillats incolores réunis montre que le taux de transformation moyen est de 92 % et que le rendement moyen en prénal est de 85,4 % par rapport au méthylbutynol transformé.

Le rendement moyen en prénal, pour les trois opérations, est de 78,6 % par rapport au méthylbutynol mis en oeuvre.

EXEMPLE 2

Dans un ballon tricol de 25 cm3 muni d'un réfrigérant, d'une arrivée d'argon, d'un septum pour prélèvement d'échantillon et d'une agitation magnétique, on introduit, sous atmosphère inerte :

- méthylbutynol : 4,34 g (51,6 m.moles)
- acétate d'éthyle : 4,5 g (51,1 m.moles)
- titanate de butyle : 0,5 g (1,46 m.moles)

On chauffe pendant 4 heures à une température comprise entre 80 et 90° C. L'analyse par chromatographie en phase vapeur montre l'absence de formation de prénal. On ajoute alors 0,5 g de chlorure cuivreux (5,05 m.moles). Après 2 heures de chauffage à 90° C, l'examen par chromatographie en phase vapeur en surface montre que le taux de transformation du méthylbutynol est de 80 % environ et que le rendement en prénal est de 65 % par rapport au méthylbutynol transformé.

EXEMPLE 3

On opère comme dans l'exemple 2 mais en absence de titanate de butyle.

Après 1 heure 30 minutes de chauffage à 90° C, le méthylbutynol est récupéré inchangé.

EXEMPLE 4

On opère comme dans l'exemple 2, mais en utilisant :

- méthylbutynol : 4,34 g (51,6 m.moles)
- benzoate de méthyle : 5,43 g (39,8 m.moles)
- titanate de butyle : 0,25 g (0,73 m.moles)

Après 3 heures de chauffage à 130° C, on n'observe pas la formation de prénal.

On ajoute alors 0,1 g de chlorure cuivreux (1,01 m.mole) puis on chauffe pendant 2 heures à 130° C.

L'examen par chromatographie en phase vapeur en surface montre que le taux de transformation du méthylbutynol est voisin de 100 % et que le rendement en prénal est de 58 %.

EXEMPLE 5

On opère comme dans l'exemple 4, mais en utilisant :

- méthylbutynol : 4,34 g (51,6 m.moles)
- phosphate de tributyle : 4,9 g (18,4 m.moles)
- titanate de butyle : 0,25 g (0,73 m.mole)
- chlorure cuivreux : 0,1 g (1,01 m.mole)

Après 2 heures de chauffage à 130° C, l'analyse chromatographique montre que le taux de transformation du méthylbutynol est voisin de 100 % et que le rendement en prénal est voisin de 60 %.

EXEMPLE 6

On opère comme dans l'exemple 5, mais en absence de chlorure cuivreux. Après 2 heures de chauffage à 140° C, on n'observe pas la formation de prénal.

EXEMPLE 7

On opère comme dans l'exemple 2, mais en utilisant :

- méthylbutynol : 4,34 g (51,6 m.moles)

- benzoate de méthyle : 0,5 g (3,68 m.moles)
- dicyclohexyle (étalon interne) : 1,75 g
- dichlorobenzène : 26 g
- titanate de butyle : 0,25 g (0,75 m.mole)
- chlorure cuivreux : 0,1 g (1,01 m.mole)

On chauffe pendant 2 heures à 120°C.

Le taux de transformation du méthylbutynol est de 98,5 % et le rendement en prénal est de 62 % par rapport au méthylbutynol transformé.

EXEMPLE 8

On opère comme dans l'exemple 2, mais en utilisant :
- méthylbutynol : 4,34 g (51,6 m.moles)
- acétoxy-2 propène : 0,3 g (3,05 m.moles)
- dicyclohexyle (étalon interne) : 1,76 g
- titanate de butyle : 0,25 g (0,73 m.mole)
- chlorure cuivreux : 0,1 g (1,01 m.mole)

On chauffe pendant 4 heures 30 minutes à 110°C.

Le taux de transformation de méthylbutynol est de 98,5 % et le rendement en prénal est de 55 % par rapport au méthylbutynol transformé.

EXEMPLE 9

On opère comme dans l'exemple 2, mais en utilisant :
- méthylbutynol : 4,34 g (51,6 m.moles)
- acétate de butyle : 4,38 g (37,7 m.moles)
- butanol : 0,41 g (5,5 m.moles)
- dicyclohexyle (étalon interne) : 1,74 g
- titanate de butyle : 0,25 g (0,73 m.mole)
- chlorure cuivreux : 0,1 g (1,01 m.mole)

On chauffe pendant 2 heures 30 minutes à 120°C.

Le taux de transformation de méthylbutynol est de 98,5 % et le rendement en prénal est de 61 % par rapport au méthylbutynol transformé.

EXEMPLE 10

On opère comme dans l'exemple 2, mais en utilisant :
- méthylbutynol : 4,36 g (51,9 m.moles)
- benzoate de méthyle : 5,44 g (40 m.moles)
- triphénylsilanol : 0,43 g (1,55 m.moles)
- dicyclohexyle (étalon interne) : 1,76 g
- titanate de butyle : 0,29 g (0,86 m.mole)
- chlorure cuivreux : 0,11 g (1,1 m.mole)

On chauffe pendant 2 heures 30 minutes à 130°C.

Le taux de transformation du méthylbutynol est de 96 % et le rendement en prénal est de 60 % par rapport au méthylbutynol transformé.

EXEMPLE 11

On opère comme dans l'exemple 2, mais en utilisant :
- méthylbutynol : 4,35 g (51,8 m.moles)
- acétate de méthylbutynol : 0,51 g (4,04 m.moles)
- dicyclohexyle (étalon interne) : 1,75 g
- dichlorobenzène : 13,10 g
- titanate de butyle : 0,25 g (0,75 m.mole)
- chlorure cuivreux : 0,101 g (1,02 m.mole)

On chauffe pendant 1 heure 30 minutes à 130°C.

Le taux de transformation du méthylbutynol est de 97 % et le rendement en prénal est de 61 % par

rapport au méthylbutynol transformé.

EXEMPLE 12

On opère comme dans l'exemple 2, mais en utilisant :
- méthylbutynol : 4,34 g (51.6 m.moles)
- benzoate de méthyle : 5,42 g (39,8 m.moles)
- acide acétique : 0,525 g (8,74 m.moles)
- dicyclohexyle (étalon interne) : 1,73 g
- titanate de butyle : 0,25 g (0,73 m.mole)
- chlorure cuivreux : 0,1 g (1,01 m.mole)

On chauffe pendant 2 heures à 130 °C.

Le taux de transformation du méthylbutynol est de 99 % et le rendement en prénal est de 67 % par rapport au méthylbutynol transformé.

EXEMPLE 13

On opère comme dans l'exemple 2, mais en utilisant :
- méthylbutynol : 4,34 g (51,6 m.moles)
- acide acétique : 0,525 g (8,74 m.moles)
- dicyclohexyle (étalon interne) : 1,728 g
- dichlorobenzène : 6,55 g
- titanate de butyle : 0,25 g (0,73 m.mole)

Après 2 heures de chauffage à 130 °C, on n'observe pas la formation de prénal.

On ajoute alors 0,1 g de chlorure cuivreux (1,01 m.mole) puis on chauffe à 130 °C pendant 1 heure 30 minutes.

Le taux de transformation du méthylbutynol est de 92 % et le rendement en prénal est de 78 % par rapport au méthylbutynol transformé.

EXEMPLE 14

On opère comme dans l'exemple 13, mais en absence de titanate de butyle.

Après 2 heures de chauffage à 130 °C, on n'observe pas la formation de prénal.

EXEMPLE 15

On opère comme dans l'exemple 2, mais en utilisant :
- méthylbutynol : 4,34 g (51,6 m.moles)
- anhydride acétique : 0,324 g (3,17 m.moles)
- dichlorobenzène : 6,55 g
- dicyclohexyle (étalon interne) : 1,73 g
- titanate de butyle : 0,25 g (0,73 m.mole)
- chlorure cuivreux : 0,1 g (1,01 m.mole)

Après 30 minutes de chauffage à 130 °C, le taux de transformation du méthylbutynol est de 88 % et le rendement en prénal est de 80 % par rapport au méthylbutynol transformé.

Après 1 heure 30 minutes de chauffage à 130 °C, le taux de transformation du méthylbutynol est voisin de 100 % et le rendement en prénal est de 66 % par rapport au méthylbutynol transformé.

EXEMPLE 16

On opère comme dans l'exemple 2, mais en utilisant :
- méthylbutynol : 4,34 g (51,6 m.moles)
- acide benzoïque : 1 g (8,19 m.moles)
- dicyclohexyle (étalon interne) : 1,73 g
- dichlorobenzène : 6,55 g
- titanate de butyle : 0,25 g (0,73 m.mole)
- chlorure cuivreux : 0,1 g (1,01 m.mole)

Après 1 heure 30 minutes de chauffage à 130 °C, le taux de transformation du méthylbutynol est de 95

% et le rendement en prénal est de 79 % par rapport au méthylbutynol transformé.

EXEMPLE 17

On opère comme dans l'exemple 2, mais en utilisant :
- méthylbutynol : 4,34 g (51,6 m.moles)
- acide téréphtalique : 0,8 g (4,81 m.moles)
- dicyclohexyle (étalon interne) : 1,73 g
- dichlorobenzène : 6,55 g
- titanate de butyle : 0,25 g (0,73 m.mole)
- chlorure cuivreux : 0,1 g (1,01 m.mole)

Après 1 heure de chauffage à 130 °C, le taux de transformation du méthylbutynol est de 98 % et le rendement en prénal est de 76 % par rapport au méthylbutynol transformé.

EXEMPLE 18

On opère comme dans l'exemple 2, mais en utilisant :
- méthylbutynol : 4,34 g (51,6 m.moles)
- acide méthyl-4 benzoïque : 1,2 g (8,81 m.moles)
- dicyclohexyle (étalon interne) : 1,73 g
- dichlorobenzène : 6,55 g
- titanate de butyle : 0,25 g (0,73 m.mole)
- chlorure cuivreux : 0,1 g (1,01 m.mole)

On chauffe à 130 °C et on suit l'évolution du mélange réactionnel par chromatographie en phase vapeur :
- après 30 minutes, le taux de transformation du méthylbutynol est de 94 % et le rendement en prénal est de 91 % par rapport au méthylbutynol transformé,
- après 1 heure, le taux de transformation du méthylbutynol est de 97 % et le rendement en prénal est de 89 % par rapport au méthylbutynol transformé,
- après 1 heure 30 minutes, le taux de transformation du méthylbutynol est de 98 % et le rendement en prénal est de 86 % par rapport au méthylbutynol transformé.

EXEMPLE 19

On opère comme dans l'exemple 2, mais en utilisant :
- méthylbutynol : 4,34 g (51,6 m.moles)
- acide hexanoïque : 1,02 g (8,78 m.moles)
- dicyclohexyle (étalon interne) : 1,73 g
- dichlorobenzène : 6,55 g
- titanate de butyle : 0,25 g (0,73 m.mole)
- chlorure cuivreux : 0,1 g (1,01 m.mole)

On chauffe à 130 °C et on suit l'évolution du mélange réactionnel par chromatographie en phase vapeur :
- après 30 minutes, le taux de transformation du méthylbutynol est de 87 % et le rendement en prénal est de 91 % par rapport au méthylbutynol transformé,
- après 1 heure, le taux de transformation du méthylbutynol est de 95 % et le rendement en prénal est de 86 % par rapport au méthylbutynol transformé,
- après 1 heure 30 minutes, le taux de transformation du méthylbutynol est de 97 % et le rendement en prénal est de 84 % par rapport au méthylbutynol transformé.

EXEMPLE 20

On opère comme dans l'exemple 2, mais en utilisant :
- méthylbutynol : 4,34 g (51,6 m.moles)
- acide adipique : 0,65 g (4,45 m.moles)
- dicyclohexyle (étalon interne) : 1,73 g
- dichlorobenzène : 6,55 g
- titanate de butyle : 0,25 g (0,73 m.mole)

- chlorure cuivreux : 0,1 g (1,01 m.mole)

Après 4 heures de chauffage à 130° C, le taux de transformation du méthylbutynol est de 82 % et le rendement en prénal est de 74 % par rapport au méthylbutynol transformé.

EXEMPLE 21

On opère comme dans l'exemple 2, mais en utilisant :

- déhydrolinalol : 4 g (26,3 m.moles)
- acétate de butyle : 4,375 g (37,7 m.moles)
- dicylohexyle (étalon interne) : 1,72 g
- titanate de butyle : 0,13 g (0,38 m.mole)
- chlorure cuivreux : 0,1 g (1,01 m.mole)

Après 3 heures de chauffage à 130° C, le taux de transformation du déhydrolinalol est de 93 % et le rendement en citral est de 45 % par rapport au déhydrolinalol transformé.

EXEMPLE 22

On opère comme dans l'exemple 2, mais en utilisant :

- méthylbutynol : 4,34 g (51,6 m.moles)
- dichlorobenzène : 6,55 g
- dicyclohexyle (étalon interne) : 1,73 g
- titanate de butyle : 0,25 g (0,73 m.mole)
- chlorure cuivreux : 0,10 g (1,01 m.mole)

Après 1 heure 30 minutes de chauffage à 130° C, le taux de transformation du méthylbutynol est de 99 % et le rendement en prénal est de 60 % par rapport au méthylbutynol transformé.

EXEMPLE 23

On opère comme dans l'exemple 2, mais en utilisant :

- méthylbutynol : 4,34 g (51,6 m.moles)
- acide méthyl-4 benzoïque : 1,2 g (8,81 m.moles)
- dicylohexyle (étalon interne) : 3,46 g
- dichlorobenzène : 13,1 g
- dibutoxy-bis-acétylacétonate de titane : 0,3 g (0,72 m.mole)
- chlorure cuivreux : 0,10 g (1,01 m.mole)

Après 2 heures de chauffage à 130° C, le taux de transformation du méthylbutynol est de 98 % et le rendement en prénal est de 88 % par rapport au méthylbutynol transformé.

EXEMPLE 24

On opère comme dans l'exemple 2, mais en utilisant :

- méthylbutynol : 4,34 g (51,6 m.moles)
- acide méthyl-4 benzoïque : 1,2 g (8,81 m.moles)
- dicyclohexyle (étalon interne) : 1,73 g
- dichlorobenzène : 6,55 g
- titanate de butyle : 0,25 g (0,73 m.mole)
- chlorure cuivrique : 0,15 g (1,12 m.mole)

Après 1 heure 30 minutes de chauffage à 130° C, le taux de transformation du méthylbutynol est de 97 % et le rendement en prénal est de 77 % par rapport au méthylbutynol transformé.

EXEMPLE 25

On opère comme dans l'exemple 2, mais en utilisant :

- méthylbutynol : 4,34 g (51,6 m.moles)
- acide méthyl-4 benzoïque : 1,2 g (8,81 m.moles)
- dicyclohexyle (étalon interne) : 1,73 g
- dichlorobenzène : 6,55 g
- titanate de butyle : 0,25 g (0,73 m.mole)

- trifluoroacétate d'argent : 0,22 g (1,0 m.mole)

Après 2 heures de chauffage à 130° C, le taux de transformation du méthylbutynol est de 61 % et le rendement en prénal est de 82 % par rapport au méthylbutynol transformé.

EXEMPLE 26

On opère comme dans l'exemple 2, mais en utilisant :

- triméthyl-2,2,6 éthynyl-1 cyclohexanol-1 : 9,2 g (55,3 m.moles)
- acide méthyl-4 benzoïque : 1,2 g (8,81 m.moles)
- dichlorobenzène : 13,1 g
- dicyclohexyle (étalon interne) : 3,5 g
- titanate de butyle : 0,25 g (0,73 m.mole)
- chlorure cuivreux : 0,10 g (1,01 m.mole)

Après chauffage à 130° C pendant 4 heures, le taux de transformation du triméthyl-2,6,6 éthynyl-1 cyclohexanol-1 est de 96 %.

On obtient, avec un rendement quantitatif, un mélange de (triméthyl-2,2,6 cyclohexylidène) formaldéhyde (I) et de (triméthyl-2,6,6 cyclohexène-1 yl)-acétaldéyde (II) dans le rapport molaire 0,34.

Le (triméthyl-2,2,6 cyclohexylidène) formaldéhyde peut être isomérisé en (triméthyl-2,6,6 cyclohexène-1 yl)-acétaldéhyde.

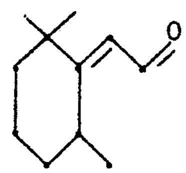

(I)

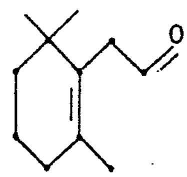

(II)

EXEMPLE 27

On opère comme dans l'exemple 2, mais en utilisant :

- déhydrolinalol : 4 g (26,3 m.moles)
- acide méthyl-4 benzoïque : 0,6 g (4,41 m.moles)
- dichlorobenzène : 6,55 g
- carbure en $C_{20}$ (étalon interne) : 1,70 g
- titanate d'éthyle : 0,09 g (0,39 m.mole)
- chlorure cuivreux : 0,10 g (1,01 m.mole)

Après 1 heure 30 minutes de chauffage à 125° C, le taux de transformation du déhydrolinalol est de 98 %. On obtient ainsi un mélange de néral et de géranial (rapport = 0,65) avec un rendement de 65 % par rapport au déhydrolinalol transformé.

EXEMPLE 28

Dans un ballon tricol de 25 cm3 muni d'un réfrigérant, d'une arrivée d'argon, d'un septum pour prélèvement d'échantillon et d'une agitation magnétique, on introduit, sous atmosphère inerte :

- acide méthyl-4 benzoïque : 2,4 g (17,63 m.moles)
- dichlorobenzène : 13,1 g
- dicylohexyle (étalon interne) : 3,46 g

Dans le mélange hétérogène à 20° C, on ajoute

- tétrachlorure de titane : 0,28 g (1,46 m.mole)

La température monte à 25° C et le mélange réactionnel se colore en jaune vif. On chauffe progressivement jusqu'à 100° C et maintient à cette température pendant 30 minutes sous balayage léger d'argon pour éliminer l'acide chlorhydrique formé. Le mélange réactionnel, qui est de couleur orange-marron, est homogène. Après refroidissement à 40° C, on introduit :

- méthylbutynol : 8,68 g (103,2 m.moles)

- chlorure cuivreux : 0,2 g (2,02 m.moles)

Après 30 minutes de chauffage à 130 °C, le taux de transformation du méthylbutynol est de 99 % et le rendement en prénal est de 80 % par rapport au méthylbutynol transformé.

EXEMPLE 29

On opère comme dans l'exemple 2, mais en utilisant :

- méthylbutynol : 8,68 g (103,2 m.moles)
- acide méthyl-4 benzoïque : 2,4 g (17,6 m.moles)
- dichlorobenzène : 13,1 g
- dicyclohexyle (étalon interne) : 3,46 g
- acétylacétonate de titanyle : 0,4 g (1,53 m.mole)
- chlorure cuivreux : 0,2 g (2,02 m.moles)

Après 1 heure 30 minutes de chauffage à 130 °C, le taux de transformation du méthylbutynol est de 99 % et le rendement en prénal est de 85 % par rapport au méthylbutynol transformé.

EXEMPLE 30

On opère comme dans l'exemple 2, mais en utilisant :

- méthylbutynol : 8,68 g (103,2 m.moles)
- dichlorobenzène : 13,1 g
- dicyclohexyle (étalon interne) : 3,46 g
- acide méthyl-4 benzoïque : 2,4 g (17,6 m.moles)
- dichlorure de titanocène : 0,38 g (1,53 m.moles)
- chlorure cuivreux : 0,2 g (2,02 m.moles)

Après 2 heures de chauffage à 130 °C, le taux de transformation du méthylbutynol est de 99 % et le rendement en prénal est de 82 % par rapport au méthylbutynol transformé.

EXEMPLE 31

Dans un ballon de 100 cm3, on introduit, sous atmosphère d'argon :

- acide méthyl-4 benzoïque : 2,4 g (17,6 m.moles)
- acide acétique : 5,25 g
- dichloroéthane : 50 cm3
- trichlorure de titane [en solution aqueuse à 15 % (p/v)] : 2,76 g (2,24 m.moles de $TiCl_3$)

On chauffe à 85 °C et distille le mélange azéotropique dichloroéthane-eau. Le mélange réactionnel, initialement mauve et hétérogène, vire au violet-noir et devient homogène.

On ajoute alors :

- dichlorobenzène : 10 cm3
- chlorure cuivrique anhydre : 0,3 g (2,23 m.moles)

On chauffe au reflux en éliminant le dichloroéthane résiduel par distillation. La température du mélange réactionnel atteint 120 °C. Le mélange réactionnel se décolore progressivement en passant du violet-noir au brun, au vert, au jaune puis au blanc. On observe la formation d'un précipité. Après refroidissement, on ajoute :

- dicyclohexyle (étalon interne) : 3,46 g
- méthylbutynol : 8,68 g (103,2 m.moles)

On chauffe. Le précipité blanc se dissout. La température du reflux s'établit à 115 °C et le mélange réactionnel jaune clair devient homogène. La température est ensuite portée rapidement à 130 °C et on chauffe à cette température pendant 1 heure 30 minutes.

Le taux de transformation du méthylbutynol est de 98 % et le rendement en prénal est de 84 % par rapport au méthylbutynol transformé.

EXEMPLE 32

On opère comme dans l'exemple 2, mais en utilisant :

- méthylbutynol : 4,34 g (51,6 m.moles)
- benzonitrile : 5,0 g

- dicyclohexyle (étalone interne) : 1,73 g
- acide méthyl-4 benzoïque : 1,2 g (8,81 m.moles)
- titanate de butyle : 0,25 g (0,73 m.mole)
- chlorure cuivreux : 0,073 g (0,74 m.mole)

Après 1 heure 30 minutes de chauffage à 130° C, le taux de transformation du méthylbutynol est de 98 % et le rendement en prénal est de 81 % par rapport au méthylbutynol transformé.

EXEMPLE 33

On opère comme dans l'exemple 2 mais en utilisant :

- méthylbutynol : 8,68 g (103 m.moles)
- dichlorobenzène : 10 cm3
- dicyclohexyle (étalon interne) : 4 cm3
- acide méthoxy-4 benzoïque : 2,7 g (17,76 m.moles)
- titanate de butyle : 0,56 g (1,67 m.mole)
- chlorure cuivreux : 0,2 g (2,02 m.moles)

Après 50 minutes de chauffage à 130° C, le taux de transformation du méthylbutynol est de 96,4 % et le rendement en prénal est de 85,8 % par rapport au méthylbutynol transformé.

EXEMPLE 34

On opère comme dans l'exemple 2 mais en utilisant :

- méthylbutynol : 8,68 g (103 m.moles)
- dichlorobenzène : 10 cm3
- dicyclohexyle (étalon interne) : 4 cm3
- acide phénoxy-4 benzoïque : 3,8g (17,76 m.moles)
- titanate de butyle : 0,56 g (1,67 m.mole)
- chlorure cuivreux : 0,2 g (2,02 m.moles)

Après 1 heure 30 minutes de chauffage à 130° C, le taux de transformation du méthylbutynol est de 93,8 % et le rendement en prénal est de 82,3 % par rapport au méthylbutynol transformé.

EXEMPLE 35

On opère comme dans l'exemple 2 mais en utilisant :

- méthybutynol : 0,1 mole
- dichlorobenzène : 25,4 g
- acide octanoïque : 0,0037 mole
- titanate de butyle : 0,00073 mole
- chlorure cuivreux : 0,00081 mole

Après 3 heures de chauffage à 130° C, le taux de transformation du méthylbutynol est de 97 % et le rendement en prénal est de 80 % par rapport au méthylbutynol transformé.

EXEMPLE 36

On opère comme dans l'exemple 2 mais en utilisant :

- méthylbutynol : 0,1 mole
- dichlorobenzène : 25,4 g
- acide phénylacétique : 0,0037 mole
- titanate de butyle : 0,00073 mole
- chlorure cuivreux : 0,00081 mole

Après 3 heures de chauffage à 130° C, le taux de transformation du méthylbutynol est de 85 % et le rendement en prénal est de 82 % par rapport au méthylbutynol transformé.

EXEMPLE 37

On opère comme dans l'exemple 2 mais en utilisant :

- méthylbutynol : 0,1 mole
- dichlorobenzène : 25,4 g

- acide éthyl-2 hexanoïque : 0,0074 mole
- titanate de butyle : 0,00073 mole
- chlorure cuivreux : 0,00081 mole

Après 3 heures de chauffage à 130 °C, le taux de transformation du méthylbutynol est de 95 % et le rendement en prénal est de 79 % par rapport au méthylbutynol transformé.

EXEMPLE 38

On opère comme dans l'exemple 2 mais en utilisant :

- méthylbutynol : 0,1 mole
- dichlorobenzène : 25,4 g
- acide heptanoïque : 0,0074 mole
- titanate de butyle : 0,00073 mole
- chlorure cuivreux : 0,00081 mole

Après 2 heures 30 minutes de chauffage à 130 °C, le taux de transformation du méthylbutynol est de 96 % et le rendement en prénal est de 85 % par rapport au méthylbutynol transformé.

EXEMPLE 39

On opère comme dans l'exemple 2 mais en utilisant :

- méthylbutynol : 8,4 g (99,86 m.moles)
- benzoate de méthyle : 17,9 g
- dicyclohexyle (étalon interne) : 4 g
- acide heptanoïque : 0,96 g (7,37 m.moles)
- titanate d'isopropyle : 0,21 g (0,75 m.mole)
- chlorure cuivreux : 0,08 g (0,81 m.mole)

Après 3 heures de chauffage à 130 °C, le taux de transformation du méthylbutynol est de 94 % et le rendement en prénal est de 86 % par rapport au méthylbutynol transformé.

EXEMPLE 40

On opère comme dans l'exemple 2 mais en utilisant :

- méthylbutynol : 8,4 g (99,86 m.moles)
- anisole : 15 g
- dicyclohexyle (étalon interne) : 4 g
- acide heptanoïque : 0,96 g (7,37 m.moles)
- titanate d'isopropyle : 0,21 g (0,75 m.mole)
- chlorure cuivreux : 0,08 g (0,81 m.mole)

Après 3 heures de chauffage à 130 °C le taux de transformation du méthylbutynol est de 97 % et le rendement en prénal est de 87 % par rapport au méthylbutynol transformé.

EXEMPLE 41

On opère comme dans l'exemple 2 mais en utilisant :

- méthylbutynol : 8,4 g (99,86 m.moles)
- phénétole : 15 g
- dicyclohexyle (étalon interne) : 4g
- acide heptanoïque : 0,96 g (7,37 m.moles)
- titanate d'isopropyle : 0,21 g (0,75 m.mole)
- chlorure cuivreux : 0,08 g (0,81 m.mole)

Après 3 heures de chauffage à 130 °C, le taux de transformation du méthylbutynol est de 96 % et le rendement en prénal est de 87 % par rapport au méthylbutynol transformé.

EXEMPLE 42

On opère comme dans l'exemple 2 mais en utilisant :

- méthylbutynol : 8,4 g (99,86 m.moles)
- dicyclohexyle (solvant) : 12,4 g

- tétradécane (étalon interne) : 4 g
- acide heptanoïque : 0,96 g (7,37 m.moles)
- titanate d'isopropyle : 0,21 g (0,75 m.mole)
- chlorure cuivreux : 0,08 g (0,81 m.mole)

Après 4 heures 45 minutes de chauffage à 130 °C, le taux de transformation du méthylbutynol est de 77 % et le rendement en prénal est de 95 % par rapport au méthylbutynol transformé.

EXEMPLE 43

Dans des tubes en verre (tubes de Carius) de 34 cm3 on introduit 21 cm3 d'un mélange constitué de :
- méthylbutynol : 5,6 g (66,6 m.moles)
- dichlorobenzène : 14,25 g
- dicyclohexyle (étalon interne) : 2,66 g
- acide méthyl-4 benzoïque : 2,45 m.moles
- titanate d'isopropyle : 0,49 m.mole
- chlorure cuivreux : 0,54 m.mole

Les tubes sont scellés sous atmosphère inerte puis chauffés dans un four à 130 °C.

Après 2 heures 30 minutes de chauffage, le taux de transformation du méthylbutynol est de 98 % et le rendement en prénal est de 90 % par rapport au méthylbutynol transformé.

EXEMPLE 44

On opère comme dans l'exemple 2 mais en utilisant :
- méthylbutynol : 8,68 g (103,2 m.moles)
- dichlorobenzène : 13,1 g
- dicyclohexyle (étalon interne) : 3,46 g
- acide méthyl-4 benzoïque : 2,4 g (17,63 m.moles)
- titanate de butyle : 0,5 g (1,47 m.mole)
- chlorure cuivreux : 0,15 g (1,52 m.mole)

Après 1 heure de chauffage à 130 °C, le taux de transformation du méthylbutynol est de 97 % et le rendement en prénal est de 85 % par rapport au méthylbutynol transformé.

EXEMPLE 45

On opère comme dans l'exemple 2 mais en utilisant :
- méthylbutynol : 8,68 g (103,2 m.moles)
- dichlorobenzène : 26,2 g
- dicyclohexyle (étalon interne) : 3,46 g
- acide méthyl-4 benzoïque : 0,6 g (4,41 m.moles)
- titanate de butyle : 0,25 g (0,73 m.mole)
- chlorure cuivreux : 0,08 g (0,81 m.mole)

Après 2 heures 30 minutes de chauffage à 130 °C, le taux de transformation du méthylbutynol est de 98 % et le rendement en prénal est de 89 % par rapport au méthylbutynol transformé.

EXEMPLE 46

On opère comme dans l'exemple 2 mais en utilisant :
- méthylbutynol : 4,4 g (52,31 m.moles)
- dichlorobenzène : 6,5 g
- dicyclohexyle (étalon interne) : 1,72 g
- acide méthyl-4 benzoïque : 1,2 g (8,81 m.moles)
- titanate de butyle : 0,25 g (0,73 m.mole)
- oxalate de cuivre, hémihydraté : 0,12 g (0,75 m.mole)

Après 5 heures de chauffage à 130 °C, le taux de transformation du méthylbutynol est de 91 % et le rendement en prénal est de 85 % par rapport au méthylbutynol transformé.

L'oxalate de cuivre est préparé en milieu aqueux par addition du carbonate basique de cuivre sur l'acide oxalique. Le précipité bleu qui se forme est séparé par filtration.

EXEMPLE 47

Dans un ballon muni d'un réfrigérant ascendant, on introduit, sous atmosphère inerte :
- dichlorobenzène : 19,5 g
- acide méthyl-4 benzoïque : 1,8 g (13,2 m.moles)
- titanate de butyle : 0,37 g (1,09 m.mole)

On chauffe pendant 15 minutes à 130 °C. Le butanol libéré se condense dans le réfrigérant. Le butanol est distillé sous pression réduite (T = 32 °C, p = 13 mm de mercure, 1,43 kPa) en chauffant le ballon jusqu'à 64 °C. On recueille ainsi un mélange de butanol (90 % de la théorie) et de dichlorobenzène (2 cm3).

On introduit alors dans le ballon :
- dichlorobenzène : 2 cm3
- méthylbutynol : 12,8 g (152,2 m.moles)
- dicyclohexyle (étalon interne) : 5,3 g
- chlorure cuivreux : 0,11 g (1,11 m.mole)

Après 2 heures de chauffage à 130 °C, le taux de transformation du méthylbutynol est de 97 % et le rendement en prénal est de 88 % par rapport au méthylbutynol transformé.

EXEMPLE 48

On opère comme dans l'exemple 2 mais en utilisant :
- méthylbutynol : 8,4 g (99,86 m.moles)
- benzoate de méthyle : 16,2 g
- dicyclohexyle (étalon interne) : 4 g
- acide crotonique : 0,4 g (4,65 m.moles)
- titanate d'isopropyle : 0,21 g (0,75 m.mole)
- chlorure cuivreux : 0,08 g (0,81 m.mole)

Après 1 heure de chauffage à 130 °C, le taux de transformation du méthylbutynol est de 96 % et le rendement en prénal est de 93 % par rapport au méthylbutynol transformé.

EXEMPLE 49

On opère comme dans l'exemple 2 mais en utilisant :
- méthylbutynol : 8,4 g (99,86 m.moles)
- nitrobenzène : 17,8 g
- dicyclohexyle (étalon interne) : 4 g
- acide heptanoïque : 0,96 g (7,37 m.moles)
- titanate d'isopropyle : 0,21 g (0,75 m.moles)
- chlorure cuivreux : 0,08 g (0,81 m.mole)

Après 2 heures de chauffage à 130 °C, le taux de transformation du méthylbutynol est de 94 % et le rendement en prénal est de 86 % par rapport au méthylbutynol transformé.

EXEMPLE 50

On opère comme dans l'exemple 2 mais en utilisant :
- méthylbutynol : 8,4 g (99,86 m.moles)
- N-méthylpyrrolidone : 15,3 g
- dicyclohexyle (étalon interne) : 4 g
- acide heptanoïque : 0,96 g (7,37 m.moles)
- titanate d'isopropyle : 0,21 g (0,75 m.moles)
- chlorure cuivreux : 0,08 g (0,81 m.mole)

Après 2 heures de chauffage à 130 °C, le taux de transformation du méthylbutynol est de 81 % et le rendement en prénal est de 65 % par rapport au méthylbutynol transformé.

EXEMPLE 51

On opère comme dans l'exemple 2 mais en utilisant :
- méthylbutynol : 8,4 g (99,86 m.moles)

- cyclohexanone : 14 g
- dicyclohexyle (étalon interne) : 4 g
- acide heptanoïque : 0,96 g (7,37 m.moles)
- titanate d'isopropyle : 0,21 g (0,75 m.mole)
- chlorure cuivreux : 0,08 g (0,81 m.mole)

Après 2 heures de chauffage à 130 °C, le taux de transformation du méthylbutynol est de 51 % et le rendement en prénal est de 63 % par rapport au méthylbutynol transformé.

EXEMPLE 52

On opère comme dans l'exemple 2 mais en utilisant :
- diméthyl-3,7 hydroxy-3 méthoxy-7 octyne-1 (méthoxydéhydrolinalol) à 95 % : 9,7 g (50 m.moles)
- benzoate de méthyle : 7,0 g
- dichlorobenzène (étalon interne) : 4,5 g
- acide crotonique : 0,2 g (2,323 m.moles)
- titanate de butyle : 0,13 g (0,382 m.mole)
- chlorure cuivreux : 0,04 g (0,404 m.mole)

Après 2 heures de chauffage à 130 °C, le taux de transformation du méthoxydéhydrolinalol est de 98 % et le rendement en diméthyl-3,7 méthoxy-7 octène-2 al est de 72 % par rapport au méthoxydéhydrolinalol transformé.

EXEMPLE 53

On opère comme dans l'exemple 2 mais en utilisant :
- phénoxy-1 hydroxy-4 méthyl-4 pentyne-2 à 91 % : 1,0 g (4,78 m.moles)
- benzoate de méthyle : 10 g
- acide crotonique : 0,1 g (1,16 m.mole)
- titanate de butyle : 0,08 g (0,235 m.mole)
- chlorure cuivreux : 0,05 g (0,505 m.mole)

Après 4 heures de chauffage à 170 °C, le taux de transformation de l'alcool acétylénique est de 75 % et le rendement en phénoxy-1 oxo-2 méthyl-4 pentène-3 est de 63 % par rapport à l'alcool acétylénique transformé.

EXEMPLE 54

Dans un ballon surmonté d'une colonne Vigreux suivie d'un réfrigérant et d'un récepteur, on introduit sous atmosphère d'argon :
- butyne-2 ol-1 : 1,9 g (26,57 m.moles)
- dichlorobenzène : 14 g
- dicyclohexyle (étalon interne) : 2 g
- acide crotonique : 0,28 g (3,25 m.moles)
- titanate de butyle : 0,18 g (0,53 m.mole)
- chlorure cuivreux : 0,1 g (1,04 m.mole)

On chauffe pendant 30 minutes à 130 °C puis pendant 2 heures à 140 °C. Les produits légers, entraînés par un courant d'argon, sont recueillis dans le récepteur refroidi extérieurement par un mélange acétone-carboglace. On recueille ainsi 1,46 g de distillat.

Dans le distillat et dans le pied de distillation, on dose le butyne-2 ol-1 et la méthylvinylcétone.

Le taux de transformation du butyne-2 ol-1 est de 70 % et le rendement en méthylvinylcétone est de 67 % par rapport au butyne-2 ol-1 transformé.

Le rendement en méthylvinylcétone isolée est de 61 %.

**Revendications**

**1.** Procédé de préparation d'un composé carbonylé éthylénique de formule générale :

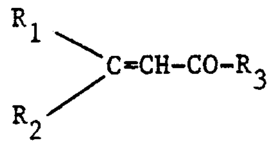

dans laquelle $R_1$, $R_2$ et $R_3$, identiques ou différents, représentent chacun un atome d'hydrogène ou un radical aliphatique saturé ou non saturé, un radical cycloaliphatique saturé ou non saturé, un radical aromatique ou un radical arylaliphatique,, ou bien $R_1$ et $R_2$ forment ensemble un radical divalent et $R_3$ représente un atome d'hydrogène ou un radical aliphatique saturé ou non saturé, un radical cycloaliphatique saturé ou non saturé, un radical aromatique ou un radical arylaliphatique, étant entendu que les radicaux aliphatiques, cycloaliphatiques, aromatiques ou arylaliphatiques peuvent être substitués par un ou plusieurs substituants, identiques ou différents, choisis parmi les atomes d'halogène et les radicaux hydroxy, alcoxy, phénoxy, acyle ou acyloxy, par isomérisation d'un alcool acétylénique de formule générale :

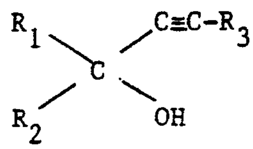

dans laquelle $R_1$, $R_2$ et $R_3$ sont définis comme précédemment caractérisé en ce que l'on chauffe en phase liquide l'alcool acétylénique en présence d'un système catalytique constitué d'un dérivé du titane et d'un dérivé du cuivre ou de l'argent, éventuellement en présence d'un ester d'acide minéral ou organique, ou d'un anhydride d'acide ou, de préférence, d'un acide organique, puis isole le composé carbonylé éthylénique obtenu.

2. Procédé selon la revendication 1 caractérisé en ce que les radicaux $R_1$, $R_2$ et $R_3$ contiennent ensemble 2 à 30 atomes de carbone et l'un au moins des radicaux $R_1$ et $R_2$ est un atome d'hydrogène ou un radical alcoyle saturé ou non saturé éventuellement substitué comportant 1 à 15 atomes de carbone.

3. Procédé selon l'une des revendications 1 ou 2 caractérisé en ce que le dérivé du titane est choisi parmi les dérivés du titane au degré d'oxydation II, III ou IV.

4. Procédé selon la revendication 3 caractérisé en ce que le dérivé du titane est choisi parmi les dérivés de formule générale $TiR_4$ dans laquelle les symboles R, identiques ou différents, représentent un atome d'halogène ou un radical OR′ ou OCOR′ dans lequel R′ représente un radical alcoyle contenant 1 à 20 atomes de carbone, ou un radical $OSiR_1R_2R_3$ dans lequel $R_1$, $R_2$ et $R_3$, identiques ou différents, représentent un radical alcoyle ou aryle, les chélates du titane, le trichlorure de titane ou le dichlorure de titanocène, les dérivés, du cyclopentadiényl-titane (IV) ou les dérivés de formule $O = T(X)_2$ dans laquelle X représente un atome d'halogène ou un radical OR′ ou OCOR′ tel que défini ci-dessus.

5. Procédé selon la revendication 3 caractérisé en ce que le dérivé du titane est choisi parmi les titanates d'alcoyle, le dibutoxy-bis-acétylacétonate de titane, le trichlorure ou le tétrachlorure de titane, l'acétylacétonate de titanyle et le dichlorure de titanocène.

6. Procédé selon l'une des revendications 1 ou 2 caractérisé en ce que le dérivé du cuivre ou de l'argent est choisi parmi les sels des acides minéraux ou organiques.

7. Procédé selon la revendication 6 caractérisé en ce que le dérivé du cuivre ou de l'argent est choisi parmi le chlorure cuivreux, le chlorure cuivrique, l'oxalate cuivrique et le trifluoroacétate d'argent.

8. Procédé selon l'une des revendications 1 ou 2 caractérisé en ce que l'on opère en présence d'un acide organique, éventuellement sous form d'ester ou d'anhydride, choisi parmi les acides ou diacides

aliphatiques saturés ou non saturés contenant 1 à 20 atomes de carbone et les acides et diacides aromatiques éventuellement substitués, d'un ester inorganique choisi parmi les esters de l'acide phosphorique ou de l'acide sulfonique.

9. Procédé selon la revendication 8 caractérisé en ce que l'on opère en présence d'un acide choisi parmi les acides acétique, hexanoïque, heptanoïque, éthyl-2 hexanoïque, octanoïque, adipique, crotonique, benzoïque, méthyl-4 benzoïque, méthoxy-4 benzoïque, phénoxy-4 benzoïque, phénylacétique ou téréphtalique.

10. Procédé selon l'une des revendications 1 ou 2 caractérisé en ce que, pour une mole d'alcool acétylénique mis en oeuvre, on utilise, 0,005 à 0,05 mole de dérivé du titane et 0,005 à 0,1 mole de dérivé du cuivre ou de l'argent.

11. Procédé selon l'une des revendications 1 ou 2 caractérisé en ce que, lorsque l'on opère en présence d'un acide ou de son dérivé ou d'un ester inorganique, on utilise 0,01 à 1 mole d'acide ou de son dérivé ou d'ester inorganique par mole d'alcool acétylénique mis en oeuvre.

12. Procédé selon l'une des revendications 1 ou 2 caractérisé en ce que l'on opère à une température comprise entre 80 et 180 °C.

13. Procédé selon l'une des revendications 1 ou 2 caractérisé en ce que l'on opère en outre dans un solvant organique choisi parmi les hydrocarbures aliphatiques, alicycliques ou aromatiques éventuellement substitués par un ou plusieurs atomes d'halogène ou radicaux alcoxy, nitro ou cyano, les amides et les cétones.

## Claims

1. A process for the preparation of an ethylenic carbonyl compound of general formula:

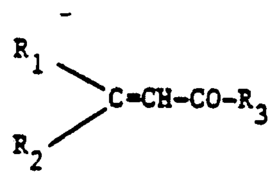

in which $R_1$, $R_2$ and $R_3$, which may be identical or different, each represent a hydrogen atom or a saturated or unsaturated aliphatic radical, a saturated or unsaturated alicyclic radical, an aromatic radical or an arylaliphatic radical or, alternatively, $R_1$ and $R_2$ together form a divalent radical and $R_3$ represents a hydrogen atom or a saturated or unsaturated aliphatic radical, a saturated or unsaturated alicyclic radical, an aromatic radical or an arylaliphatic radical, it being understood that the aliphatic, alicyclic, aromatic or arylaliphatic radicals may be substituted, if required, with one or more substituents, which may be identical or different, chosen from amongst halogen atoms and hydroxy, alkoxy, phenoxy, acyl or acyloxy radicals, by the isomerization of an acetylenic alcohol of general formula:

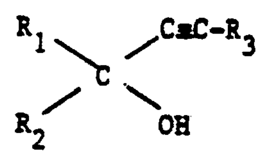

in which $R_1$, $R_2$ and $R_3$ are defined as above, wherein the acetylenic alcohol is heated in a liquid phase in the presence of a catalytic system which consists of a titanium derivative and a copper or silver derivative, if required in the presence of an inorganic or organic acid ester, or of an acid anhydride or, preferably, of an organic acid, and the ethylenic carbonyl compound obtained is isolated.

2. The process according to claim 1, wherein the radicals $R_1$, $R_2$ and $R_3$ together contain 2 to 30 carbon

atoms and at least one of the radicals $R_1$ and $R_2$ is a hydrogen atom or an optionally substituted saturated or unsaturated alkyl radical containing 1 to 15 carbon atoms.

**3.** The process according to one of claims 1 or 2, wherein the titanium derivative is chosen from amongst derivatives of titanium with a degree of oxidation of II, III or IV.

**4.** The process according to claim 3, wherein the titanium derivative is chosen from amongst derivatives of general formula $TiR_4$ in which the symbols R, which may be identical or different, represent a halogen atom or an OR' or OCOR' radical in which R' represents an alkyl radical containing 1 to 20 carbon atoms, or an $OSiR_1R_2R_3$ radical in which $R_1$, $R_2$ and $R_3$, which may be identical or different, represent an alkyl or aryl radical, chelates of titanium, titanium trichloride or titanocene dichloride, cyclopentadienyl-titanium(IV) derivatives or derivatives of formula $0 = T(X)_2$ in which X represents a halogen atom or an OR' or OCOR' radical as defined above.

**5.** The process according to claim 3, wherein the titanium derivative is chosen from amongst alkyl titanates, titanium dibutoxy-bis-acetylacetonate, titanium trichloride or tetrachloride, titanyl acetylacetonate and titanocene dichloride.

**6.** The process according to either of claims 1 or 2, wherein the copper or silver derivative is chosen from amongst the inorganic or organic acid salts.

**7.** The process according to claim 6, wherein the copper or silver derivative is chosen from amongst cuprous chloride, cupric chloride, cupric oxalate or silver trifluoroacetate.

**8.** The process according to either of claims 1 or 2, wherein the reaction is carried out in the presence of an organic acid, if required in the form of an ester or an anhydride, chosen from amongst saturated or unsaturated aliphatic acids or diacids containing 1 to 20 carbon atoms and aromatic acids and diacids, which may be substituted if required, of an inorganic ester chosen from amongst the esters of phosphoric acid or of sulphonic acid.

**9.** The process according to claim 8, wherein the reaction is carried out in the presence of an acid chosen from amongst acetic, hexanoic, heptanoic, 2-ethylhexanoic, octanoic, adipic, crotonic, benzoic, 4-methylbenzoic, 4-methoxybenzoic, 4-phenoxybenzoic, phenylacetic or terephthalic acids.

**10.** The process according to either of claims 1 or 2, wherein, for 1 mole of acetylenic alcohol employed, 0.005 to 0.05 mole of titanium derivative and 0.005 to 0.1 mole of copper or silver derivative are used.

**11.** The process according to either of claims 1 or 2, wherein, when the reaction is carried out in the presence of an acid or its derivative or an inorganic ester, 0.01 to 1 mole of acid or its derivative or of the inorganic ester is used per mole of acetylenic alcohol employed.

**12.** The process according to either of claims 1 or 2, wherein the reaction is carried out at a temperature of between 80 and 180 °C.

**13.** The process according to either of claims 1 or 2, wherein, in addition, the reaction is carried out in an organic solvent chosen from amongst aliphatic, alicyclic or aromatic hydrocarbons, which may be substituted, if required, with one or more halogen atoms or alkoxy, nitro or cyano radicals, amides and ketones.

## Patentansprüche

**1.** Verfahren zur Herstellung einer äthylenischen Carbonylverbindung der allgemeinen Formel:

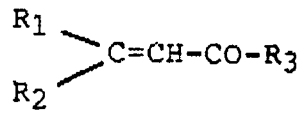

in welcher $R_1$, $R_2$ und $R_3$ unabhängig voneineinander jeweils ein Wasserstoffatom oder einen gesättigten oder ungesättigten aliphatischen Rest, einen gesättigten oder ungesättigten cycloaliphatischen Rest, einen aromatischen Rest oder einen arylaliphatischen Rest darstellen oder $R_1$ und $R_2$ zusammen einen zweiwertigen Rest darstellen und $R_3$ ein Wasserstoffatom oder einen gesättigten oder ungesättigten aliphatischen Rest, einen gesättigten oder ungesättigten cycloaliphatischen Rest, einen aromatischen Rest oder einen arylaliphatischen Rest darstellt, wobei die aliphatischen, cycloaliphatischen, aromatischen oder arylaliphatischen Reste selbstverständlich durch einen oder mehrere gleiche oder verschiedene Substituenten, ausgewählt aus den Halogenatomen und den Hydroxy-, Alkoxy-, Phenoxy-, Acyl- oder Acyloxyresten, substituiert sein können, durch Isomerisierung eines acetylenischen Alkohols der allgemeinen Formel:

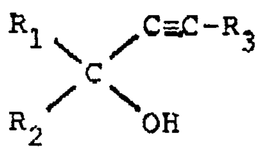

in welcher $R_1$, $R_2$ und $R_3$ die obige Bedeutung haben, dadurch gekennzeichnet, daß man den acetylenischen Alkohol in flüssiger Phase in Gegenwart eines katalytischen Systems, bestehend aus einem Titanderivat und einem Kupfer- oder Silberderivat, gegebenenfalls in Gegenwart eines Esters einer anorganischen oder organischen Säure oder eines Säureanhydrids oder, vorzugsweise, einer organischen Säure, erhitzt und dann die erhaltene äthylenische Carbonylverbindung isoliert.

2. Verfahren nach Anspruch 1, dadurch gekennzeichnet, daß die Reste $R_1$, $R_2$ und $R_3$ zusammen 2 bis 30 Kohlenstoffe enthalten und daß zumindest einer der Reste $R_1$ und $R_2$ ein Wasserstoffatom oder ein gegebenenfalls substituierter, gesättigter oder ungesättigter Alkylrest mit 1 bis 15 Kohlenstoffatomen ist,

3. Verfahren nach einem der Ansprüche 1 oder 2, dadurch gekennzeichnet, daß das Titanderivat ausgewählt ist aus den Verbindungen von Titan mit dem Oxidationsgrad II, III oder IV.

4. Verfahren nach Anspruch 3, dadurch gekennzeichnet, daß das Titanderivat ausgewählt ist aus den Verbindungen der allgemeinen Formel $TiR_4$, in welcher die Symbole R unabhängig voneinander für ein Halogenatom oder einen Rest OR' oder OCOR', worin R' einen Alkylrest mit 1 bis 20 Kohlenstoffatomen darstellt, oder einen Rest $OSiR_1R_2R_3$, worin $R_1$, $R_2$ und $R_3$ unabhängig voneinander einen Alkyl- oder Arylrest darstellen, stehen, den Titanchelaten, Titantrichlorid oder Titanocendichlorid, den Verbindungen von Cyclopentadienyltitan(IV) oder den Verbindungen der Formel $O = Ti(X)_2$, worin X ein Halogenatom oder einen Rest OR' oder OCOR', wie zuvor definiert, darstellt.

5. Verfahren nach Anspruch 3, dadurch gekennzeichnet, daß das Titanderivat ausgewählt ist aus den Alkyltitanaten, Titandibutoxy-bis-acetylacetonat, Titantrichlorid oder - tetrachlorid, Titanylacetylacetonat und Titanocendichlorid.

6. Verfahren nach einem der Ansprüche 1 oder 2, dadurch gekennzeichnet, daß das Kupfer- oder Silberderivat ausgewählt aus aus den Salzen anorganischer oder organischer Säuren.

7. Verfahren nach Anspruch 7, dadurch gekennzeichnet, daß das Kupfer- oder Silberderivat ausgewählt aus Kupfer(I)chlorid, Kupfer(II)chlorid, Kupfer(II)oxalat und Silbertrifluoracetat.

8. Verfahren nach einem der Ansprüche 1 oder 2, dadurch gekennzeichnet, daß man in Gegenwart einer organischen Säure, gegebenenfalls in Form des Esters oder Anhydrids, ausgewählt aus den gesättigten oder ungesättigten aliphatischen Säuren oder Disäuren mit 1 bis 20 Kohlenstoffatomen und den gegebenenfalls substituierten aromatischen Säuren oder Disäuren, eines anorganischen Esters, ausgewählt aus den Phosphorsäure- oder Sulfonsäureestern, arbeitet.

9. Verfahren nach Anspruch 8, dadurch gekennzeichnet, daß man in Gegenwart einer Säure, ausgewählt aus Essig-, Hexan-, Heptan-, 2-Äthylhexan-, Octan-, Adipin-, Croton-, Benzoe-, 4-Methylbenzoe-, 4-Methoxybenzoe-, 4-Phenoxybenzoe-, Phenylessig-oder Terephthalsäure, arbeitet.

10. Verfahren nach einem der Ansprüche 1 oder 2, dadurch gekennzeichnet, daß man pro ein mol eingesetztem acetylenischem Alkohol 0,005 bis 0,05 mol Titanderivat und 0,005 bis 0,1 mol Kupfer- oder Silberderivat verwendet.

11. Verfahren nach einem der Ansprüche 1 oder 2, dadurch gekennzeichnet, daß man, wenn man in Gegenwart einer Säure oder ihres Derivats oder eines anorganischen Esters arbeitet, 0,01 bis 1 mol Säure oder ihr Derivat oder anorganischen Ester pro mol des eingesetzten acetylenischen Alkohols verwendet.

12. Verfahren nach einem der Ansprüche 1 oder 2, dadurch gekennzeichnet, daß man bei einer Temperatur zwischen 80 und 180 °C arbeitet.

13. Verfahren nach einem der Ansprüche 1 oder 2, dadurch gekennzeichnet, daß außerdem in einem organischen Lösungsmittel, ausgewählt aus den gegebenenfalls durch ein oder mehrere Halogenatome oder Alkyl-, Nitro- oder Cyanoreste substituierten aliphatischen, alicyclischen oder aromatischen Kohlenwasserstoffen, arbeitet.